# EUROPEAN PATENT APPLICATION

(11) **EP 4 101 921 A1**
(43) Date of publication of application: **14.12.2022**
(21) Application number: 21191872.7
(22) Date of filing: 18.08.2021
(51) Int. Cl.: C12M 1/33, B01L 3/00

(54) **METHOD AND DEVICE FOR OPENING AN EXTERNAL LAYER STRUCTURE OF CELLS USING LASER**

(30) Priority: 07.06.2021 TW 110120587
(71) Applicant: Credo Diagnostics Biomedical Pte. Ltd., Singapore 268802 (SG)
(72) Inventor: Lai, Ying-Ta, New Taipei City 221 (TW); Ou, Yu-Cheng, New Taipei City 221 (TW); Chen, Yi-Hsi, New Taipei City 221 (TW); Tsai, Chung-Wei, New Taipei City 221 (TW); Chen, Kuan-Ying, New Taipei City 221 (TW); Tsai, Ruei-Yi, New Taipei City 221 (TW); Chen, Chih-Yuan, New Taipei City 221 (TW)
(74) Representative: Straus, Alexander

(57) **Abstract**

The present disclosure relates to a method and an apparatus for opening the external layer structure of cells using laser, wherein the short pulse laser beam excited from a laser source is converged via the optical lens and concentrated at a focus, a biological sample which is fixed on the focus or moves through the focus is treated with the concentrated short pulse laser beam, so that the cell membrane or cell wall of cells in the sample is broken.

## Description

### Field of the Invention

The present disclosure relates to a method and an apparatus for opening an external layer structure of cell using laser, and more particularly, to a method and an apparatus for opening an external layer structure of cell using a short pulse laser.

### Background of the Invention

Molecular detection has advantages of rapid screening and high accuracy, and is widely applied in related fields recently. With the progress of the molecular detection, the demand for nucleic acid extraction in the related fields is increasing steadily. Generally, the nucleic acid extraction includes the release of the nucleic acid and the purification of the nucleic acid, and the former is mainly carried out by destroying cells and releasing the nucleic acid through a mechanical method, a physical method or a chemical method. The mechanical method refers to a method using mechanical forces, such as grinding, pressure, ultrasonic vibration, or the like to release the nucleic acid from the cells. The physical method refers to a method using temperature changes, such as repeatedly and rapidly freezing and thawing the cells with liquid nitrogen to break the cells and to destroy the structures of the cells. The chemical method refers to a method using reagents, chemistry or osmotic pressure difference to break the cells. However, all of the aforementioned nucleic acid releasing methods have similar disadvantages such as tedious operation, time-consuming, costly equipment, poor convenience, or the like, and also, manual operation is easy to lead to contamination and errors, which is less efficient in mass testing or production line mode testing. Therefore, it is still necessary to the related arts to provide novel cell-breaking technologies, so as to fulfill the practical requirements of the related arts.

### Summary of the Invention

This in mind, the present disclosure aims at providing an apparatus and a method for opening an external layer structure of cells using laser that may be beneficial on mass testing or production line mode testing.

This achieved by a method and an apparatus according to the independent claims. The dependent claims pertain to corresponding further developments and improvements.

As will be seen more clearly from the detailed description following below, a method for opening an external layer structure of cells using laser is provided by the present disclosure. The method includes the following steps. Firstly, a laser source which is electrically connected to a microcontroller is provided, wherein the laser source is configured to emit a short pulse laser beam. Then, an optical lens set is provided in front of the laser source, with the short pulse laser beam passing through the optical lens set. Next, a biological sample is provided, and the microcontroller is configured to control a motion state of the biological sample, with the motion state of the biological sample being a static state or a flowing state. Finally, the biological sample is processed using the short pulse laser beam by controlling its repetition rate and output power to break cell membranes or cell walls of cells in the biological sample.

As will be seen more clearly from the detailed description following below, an apparatus for opening an external layer structure of cells using laser is provided by the present disclosure. The apparatus includes a microcontroller, a laser source, a signal generator, a power supply, and a flow rate and flow volume controller. The laser device includes an optical lens set and a laser source for emitting a short pulse laser beam. The signal generator is electrically connected between the microcontroller and the laser source, wherein the signal generator is configured to receive a first signal from the microcontroller to adjust a repetition rate of the short pulse laser beam. The power supply is electrically connected between the microcontroller and the laser source, wherein the power supply is configured to receive a second signal from the microcontroller to output a current to the laser source and to control an output power of the short pulse laser beam. The flow rate and flow volume controller is electrically connected to the microcontroller, wherein the flow rate and flow volume controller is configured to receive a third signal from the microcontroller to control a motion state of a biological sample containing cells or suspected to contain cells, wherein the short pulse laser beam is converged when passing through the optical lens set, and then is focused on a focus, and the biological sample is subjected to the short pulse laser beam, so that cell membranes or cell walls of cells in the biological sample are broken.

### Brief Description of the Drawings

In the following, the invention is further illustrated by way of example, taking reference to the accompanying drawings. Thereof:
FIG. 1 is a schematic flow chart illustrating a method for opening an external layer structure of cells using laser according to a preferable embodiment in the present disclosure.
FIG. 2 is a schematic diagram illustrating a laser device for opening an external layer structure of cells according to a preferable embodiment in the present disclosure.
FIG. 3 is a schematic diagram illustrating an apparatus for opening an external layer structure of cells using laser according to a preferable embodiment in the present disclosure.
FIG. 4 is a schematic diagram illustrating a relationship between the output power of the short pulse laser beam and the cell rupture rate according to a preferable embodiment in the present disclosure.
FIG. 5 is a schematic diagram illustrating a relationship between the repetition rate of the short pulse laser beam and the cell rupture rate according to a preferable embodiment in the present disclosure.

### Detailed Description

To provide a better understanding of the presented disclosure, preferred embodiments will be described in detail. The preferred embodiments of the present disclosure are illustrated in the accompanying drawings with numbered elements. In addition, the technical features in different embodiments described in the following may be replaced, recombined, or mixed with one another to constitute another embodiment without departing from the spirit of the present disclosure.

As disclosed herein, the term "about" or "substantial" generally means within 20%, preferably within 10%, and more preferably within 5%, 3%, 2%, 1%, or 0.5% of a given value or range. Unless otherwise expressly specified, all of the numerical ranges, amounts, values and percentages disclosed herein should be understood as modified in all instances by the term "about" or "substantial". Accordingly, unless indicated to the contrary, the numerical parameters set forth in the present disclosure and attached claims are approximations that can vary as desired.

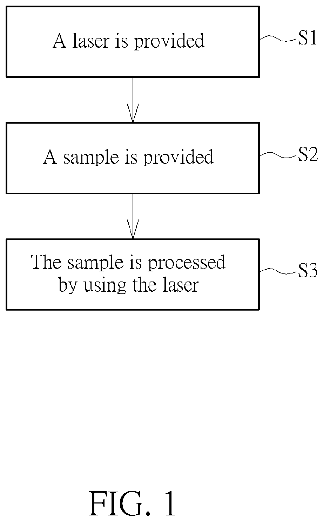

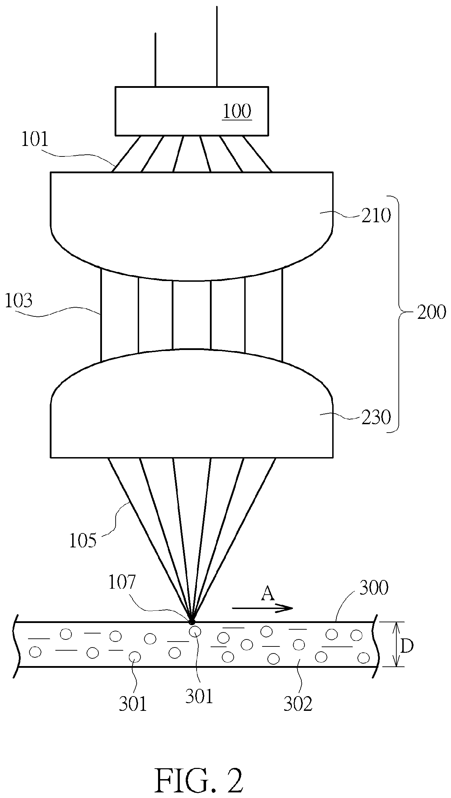

Please refer to FIG. 1, which illustrates a flow chart showing a method for opening an external layer structure of cells using laser in a first embodiment of the present disclosure. At first, a laser is provided (Step S1), and which may be a short pulse laser such as a nanosecond laser (ns-laser) and the like, but is not limited thereto. In one embodiment, a wavelength of the short pulse laser beam ranges about 800 nanometers (nm) to 1100 nm, a pulse width of the short pulse laser beam is between about 1 nanosecond (ns) and 500 ns, and a pulse energy of the short pulse laser beam ranges about 20 nanojoules (nJ) to 2000 nJ, but are not limited thereto. In a preferable embodiment, a nanosecond laser source, such as a laser source 100 as shown in FIGs. 2-3, may be optionally used to emit the short pulse laser beam, but not limited thereto. In another embodiment, the types of laser may include various lasers other than the semiconductor laser which is emitted by laser source, such as a semiconductor laser (e.g. a laser diode), a solid-state laser, a fiber laser or a combination thereof. The material of the laser source may include neodymium yttrium aluminum garnet (Nd YAG) with dual wavelengths of 808 nm and 1064 nm, indium aluminum gallium arsenide/aluminum gallium arsenide ((In)Ga(Al)As/AlGaAs) with a wavelength of 905 nm, aluminum gallium indium arsenide (InGaAlAs) with a wavelength of 980 nm, indium gallium phosphide arsenide (InGaAsP) or a combination thereof, but not limited thereto.

It is noted that, the light generated from the laser source 100 is highly directive and its energy is easy to be controlled. However, due to the larger divergence angle of the short pulse laser beam, an optical lens set 200 including a plurality of lens may be further in use in combination with the laser source 100, for improving the convergence and focusing of the light. As shown in FIGs. 2-3, the optical lens set 200 includes a light receiving lens 210 and a focusing lens 230, wherein the light receiving lens 210 may be a short-focus lens with a focus of about 5 millimeters (mm) and a numerical aperture (NA) of about 0.2 to about 0.55, so as to be beneficial on rapid convergence and enable the light nearly parallel. The focusing lens 230 may be a spherical lens, an objective lens, a non-curved lens, an aspherical lens, a diffractive optical element, or a combination thereof (e.g. aplanat lens), wherein a focus of the non-curved lens or the aspherical lens is about 11 mm and a NA thereof is about 0.2 to about 0.55, thereby focusing the light. Accordingly, while generating lights 101 from the laser source 100, the lights 101 may firstly pass through the light receiving lens 210 for light convergence, so that, lights 103 passed through the light receiving lens 210 may be parallel with each other. Then, the lights 103 may further pass through the focusing lens 230 for light-focusing, so that, lights 105, namely the short pulse laser beam, passed through the focusing lens 230 may therefor focus at a focus 107, so as to improve the efficiency and to reduce the energy loss. Preferably, the spot size of the focus 107 of the short pulse laser beam may be about 0.1 mm to about 5.1 mm in diameter, but is not limited thereto. People in the art should fully understand that the aforementioned conditions such as the focus and the NA of the light receiving lens 210 and the focusing lens 230 may all be further adjusted based on the desired spot size of the focus 107, which may be diverse according to the type and the source of the biological sample to be processed subsequently, which are not limited to what is described above. In addition, in another embodiment, the short pulse laser beam may also be emitted through various ways, for example using a laser vibrating lens or a scanning head to emit the short pulse laser beam in a comprehensive scanning manner. As an example, the laser vibrating lens may be disposed between the laser source and the optical lens set. When the biological sample is statically disposed at a fixed point (not shown in the drawings) of a flow channel 300, the laser vibrating lens may be used to emit the short pulse laser beam onto the biological sample via the optical lens set in a two-dimensional scanning manner or a three-dimensional scanning manner, wherein the fixed point may be overlapped with the focus 107.

Next, a sample is provided (as shown in Step S2 of FIG. 1), and the sample may be any biological sample 302 which may be properly processed to extract nucleic acid, such as a tissue of animal or plant, a cell of animal or plant, a microbial cell, or a sample or a specimen suspected to contain biological tissues or a cells, such as a blood sample or a body fluid sample, but is not limited thereto. Preferably, the sample may be firstly mixed with a liquid (not shown in the drawings), such as a neutral solvent like a buffer or a cleaning reagent, so that, the sample may pass through the focus 107 of the short pulse laser beam with a flow rate of about 0.01 to 1.5 milliliter per minute (ml/min), and more preferably with a flow rate of about 0.1 to 0.5 ml/min. As shown in FIG. 2, in a preferably embodiment, the biological sample 302 and the liquid are driven to flow along a specific direction "A" within a flow channel 300, so that, cells (or tissues or microorganisms) 301 in the biological sample 302 may also flow along the direction "A" accordingly. It is noted that a depth "D" of the flow channel 300 may be about 0.01 mm to 3 mm, and a width thereof (not shown in the drawings, but referring to a radial length of the flow channel 300) may also be about 0.01 mm to 3 mm, but is not limited thereto. It is also noted that the flowing mode, as well as the flow rate of the sample designed for the flow channel 300 above is only exemplary, and may be further adjusted according to the practical parameters (such as the wavelength or the pulse energy) of the short pulse laser, so as to fulfill the practical operation requirements. Furthermore, the setup conditions (such as the depth "D" or the width) of the flow channel 300 may also be diverse based on the selected optical lens set 200 and/or the size of the focus 107, and which is not limited to be above numbers.

Next, the sample is processed by using the laser (as shown in Step S3 of FIG. 1). For example, the laser is emitted on to the sample with an output power of about 10 watts (W) to 200 W, and a repetition rate about 0.1 megahertz (MHz) to 5 MHz, so that, a shock wave may be generated in the sample through the instantaneous pulse of the short pulse laser beam, thereby causing a pressure difference in cells 301 in the biological sample 302 to damage to the cell membrane or the cell wall of the cells 301 in the biological sample 302. In one embodiment, the biological sample 302 is disposed at a fixed point (not shown in the drawings, for example being overlapped with the focus 107), and the short pulse laser beam is emitted on to the biological sample 302 disposed at the fixed point. However, in another embodiment, the flow channel 300 as shown in FIG. 2 may be additionally used in combination with the short pulse laser beam, which causes an instantaneous pressure difference to the cells 301 (which flow through the focus point 107) in the biological sample 302, to damage to the cells 301. Then, the broken cells 301 are continuously passed by. Accordingly, the short pulse laser beam is available to apply on the biological sample 302 along with the flow of the biological sample 302, so as to facilitate on the mass testing or the production line mode testing.

Thus, the method for opening an external layer structure of cells using laser in the present embodiment is completed, in which, the short pulse laser beam is used to generate the instantaneous shock waves in the biological sample 302, so as to lead to a pressure difference of the cells 301 in the biological sample 302, thereby damaging the cells 301. The short pulse laser beam is preferably a nanosecond laser beam to achieve better emission performance. If the pulse width of the short pulse laser beam is too large, the short pulse laser beam may not effectively induce the pressure difference in the cells 301, which may be poor in damaging the cells 301. On the other hand, if the pulse width of the short pulse laser beam is too small, the short pulse laser beam may lead to possible damages to the operation elements such as the optical lens set 200 or the flow channel 300. In addition, the method of the present embodiment preferably includes using the flow channel 300 to transfer the biological sample 302, with the width and the depth "D" of the flow channel 300 being adjustable based on the focus of the short pulse laser beam, so that, the pulse of the short pulse laser beam may effectively generate oscillations in the cells 301 in the biological sample 302, thereby successfully damaging the cells 301. Thus, the possible failure caused by less pressure difference during processing the biological sample 302 with laser may be effectively avoided.

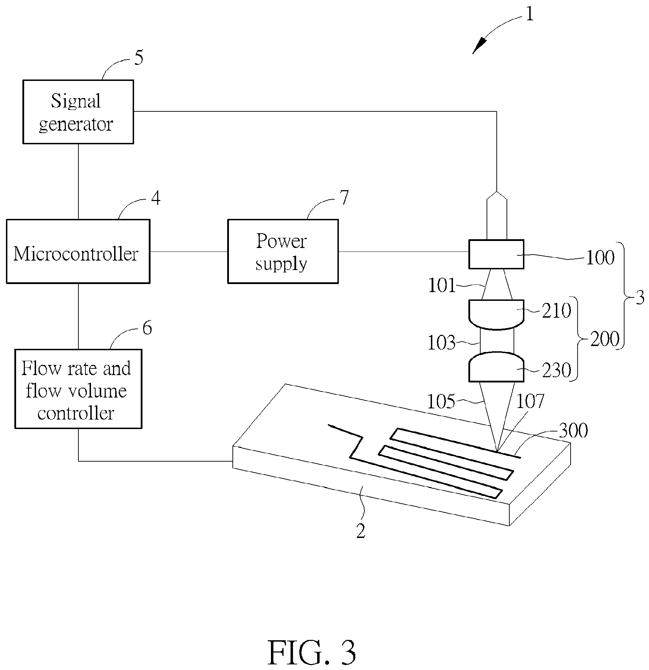

Please refer to FIG. 3, which illustrates an apparatus 1 for opening an external layer structure of cells using laser, and the apparatus 1 includes a laser device 3, a microcontroller 4, a signal generator 5, a flow rate and flow volume controller 6, and a power supply 7. Please also refer to FIG. 2, the laser device 3 as shown in FIG. 3 further includes the optical lens set 200 and the laser source 100 for emitting the short pulse laser beam. The microcontroller 4 of the apparatus 1 for opening an external layer structure of cells is configured to transmit a first signal to the signal generator 5 which is electrically connected to the microcontroller 4 and the source laser 100, and the signal generator 5 is configured to adjust a repetition rate of the short pulse laser beam based on the first signal. Then, the microcontroller 4 is configured to transmit a second signal to the power supply 7 which is connected thereto, and the power supply 7 is configured to output a current to the laser source 100 based on the second signal, so as to control the output power of the short pulse laser beam. Furthermore, the microcontroller 4 is configured to transmit a third signal to the flow rate and flow volume controller 6 which is connected thereto, and the flow rate and flow volume controller 6 is optionally coupled to an analysis cartridge 2 and which is configured to inject a biological sample 302 including the cells 301 (as shown in FIG. 2) or suspected to contain cells into the flow channel 300 of the analysis cartridge 2 based on the third signal. Also, the flow rate and flow volume controller 6 is configured to control a motion state of the biological sample 302 within the flow channel 300, for example, being at the static state or the flowing state. Then, the short pulse laser beam generated from the laser source 100 is converged by the optical lens set 200 and focused on the focus 107, and the biological sample 302 may be immediately processed by the short pulse laser beam at the focus 107, thereby breaking the cell membrane or the cell wall of the cells 301 in the sample 302.

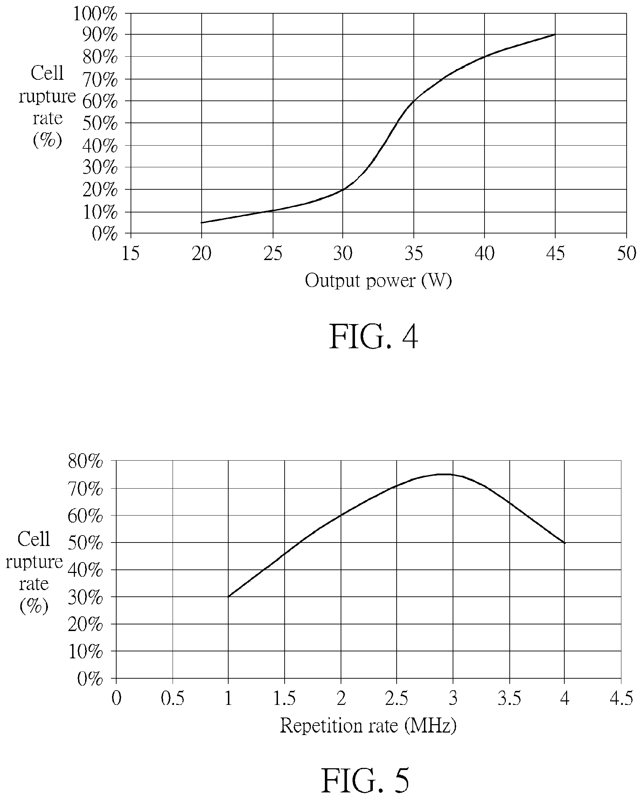

In one embodiment, the light receiving lens 210 and the focusing lens 230 of the laser device 3 have a NA value being greater than 0.2, which is beneficial on converging the short pulse laser beam, and reducing the loss of light. The flow rate and flow volume controller 6 for example includes a pump such as a syringe pump, cylinder-type power pump, quantitative liquid pump, micro gas pump or a combination thereof. When the output power (about 20 W to 45 W) of the short pulse laser beam is changed, and the repetition rate of the short pulse laser beam and the flow rate of the biological sample 302 are fixed at 2 MHz and 0.41 ml/min, respectively, the increase of the cell rupture rate of the cells 301 is positively regulated by the increase of the output power of the short pulse laser beam (as shown in FIG. 4). However, if the output power of the short pulse laser beam is lower than 30 W, the cell rupture rate of the cells 301 is dramatically decreased. Therefore, the method and apparatus 1 of the present disclosure may effectively increase the cell rupture rate of the cells 301 within the biological sample 302 to about 90%, thereby effectively extracting the nucleic acid of the cells 301 within the biological sample 302, followed by performing a nucleic acid amplification reaction using the extracted nucleic acid in the subsequent procedure.

In another embodiment, when the repetition rate is changed (about 0.1 MHz to 5 MHz) and the output power and flow rate are fixed at 40 W and 0.41 ml/min respectively, the cells 301 disposed within the flow channel 300 may not be sufficiently damaged with the fixed flow rate of biological sample 302 and the too low repetition rate of the short pulse laser beam (as shown in FIG. 5). However, the fixed flow rate of biological sample 302 and the too large repetition rate of the short pulse laser beam may reduce the pulse energy and cause the worse damage effect of the cells 301.

Therefore, the method for opening an external layer structure of cells using laser of the present disclosure may be further applied on the apparatus 1, as well as an extraction reaction by using an analysis cartridge, in the present disclosure. Accordingly, the biological sample 302 may be disposed within the analysis cartridge, followed by flowing in the analysis cartridge with a particular flow rate, and the short pulse laser beam is generated to cause a wave shock on the biological sample 302, damaging to the cells 301 within the biological sample 302. The short pulse laser beam is but not limited to be generated through the laser source 100 and the optical lens set 200 of the device 1, and the laser source 100 may also be integrated with the analysis cartridge to converge the short pulse laser beam. Through these arrangements, the method of the present disclosure may be combined with an analysis cartridge and a laser device which are commonly used in the related arts, and which is beneficial on mass testing and production line mode testing.

## Claims

1. A method for opening the external layer structure of cells using laser, **characterized by** comprising;
providing a laser source (100) which is electrically connected to a microcontroller (4), wherein the laser source (100) is configured to emit a short pulse laser beam;
providing an optical lens set (200) in front of the laser source (100), with the short pulse laser beam passing through the optical lens set (200);
providing a biological sample (302), wherein the microcontroller (4) is configured to control a motion state of the biological sample (302), and the motion state of the biological sample (301) comprises a static state or a flowing state; and
processing the biological sample (302) using the short pulse laser beam to break cell membranes or cell walls of cells (301) within the biological sample (302), wherein a repetition rate and a output power of the short pulse laser beam is controlled during processing the biological sample (302).

2. The method according to claim 1, **characterized in that** the short pulse laser beam comprises a nanosecond pulse laser beam, a pulse width of the short pulse laser beam is between 1 nanosecond (ns) and 500 ns, a wavelength of the short pulse laser beam ranges from 800 nanometers (nm) to 1100 nm, a pulse energy of the short pulse laser beam ranges from 20 nanojoules (nJ) to 2000 nJ, the output power of the short pulse laser beam is between 10 watts (W) and 200 W, and the repetition rate of the short pulse laser beam is between 0.1 megahertz (MHz) and 5 MHz.

3. The method according to any one of claims 1 to 2, **characterized in that** the motion state of the biological sample (302) is the static state, the biological sample (302) is disposed at a fixed point in a flow channel (300), and a vibrating mirror is disposed between the laser source (100) and the optical lens set (200) for emitting the short pulse laser beam onto the biological sample (302) in a two-dimensional or three-dimensional scanning mode.

4. The method according to any one of claims 1 to 2, **characterized in that** the motion state of the biological sample (302) is the flowing state, the biological sample (302) flows at a flow rate ranged between 0.01 milliliter per minute (ml/min) and 1.5 ml/min in a flow channel (300) having a depth of 0.01 millimeter (mm) to 3 mm and a width of 0.01 mm to 3 mm, and the short pulse laser beam is emitted onto the biological sample (302) along with the flow of the biological sample (302).

5. The method according to any one of claims 1 to 4, **characterized in that** the laser source (100) is selected from a group consisting of a semiconductor laser, a solid-state laser and a fiber laser, and a material of the laser source (100) is selected from a group consisting of neodymium yttrium aluminum garnet (Nd YAG), indium gallium arsenide/aluminum gallium arsenide((In)Ga(Al)As/AlGaAs), aluminum gallium indium arsenide (InGaAlAs), and indium gallium phosphide arsenide (InGaAsP).

6. The method according to any one of claims 1 to 5, **characterized in that** the optical lens set (200) is configured to converge the short pulse laser beam, and a spot size of a focus (107) of the short pulse laser beam is between 0.1 mm and 5.1 mm in diameter, and the semiconductor laser comprises a laser diode.

7. The method according to any one of claims 1 to 6, **characterized in that** the biological sample (302) comprises a tissue of animal or plant, a cell of animal or plant, a microbial cell, or a sample suspected to contain biological tissues or cells (301).

8. An apparatus (1) for opening the external layer structure of cells using laser, **characterized by** comprising:
a microcontroller (4);
a laser device (3), comprising an optical lens set (200) and a laser source (100) for emitting a short pulse laser beam;
a signal generator (5), electrically connected between the microcontroller (4) and the laser source (100), wherein the signal generator (5) is configured to receive a first signal from the microcontroller (4) to adjust a repetition rate of the short pulse laser beam;
a power supply (7), electrically connected between the microcontroller (4) and the laser source (100), wherein the power supply (7) is configured to receive a second signal from the microcontroller (4) to output a current to the laser source (100) and to control an output power of the short pulse laser beam; and
a flow rate and flow volume controller (6), electrically connected to the microcontroller (4), wherein the flow rate and flow volume controller (6) is configured to receive a third signal from the microcontroller (4) to control a motion state of a biological sample (302) comprising cells (301) or suspected to contain cells (301), wherein the short pulse laser beam is converged when passing through the optical lens set (200), and then is focused on a focus (107), and the biological sample (302) is subjected to the short pulse laser beam, so that cell membranes or cell walls of cells (301) in the biological sample (302) are broken.

9. The apparatus (1) according to claim 8, **characterized in that** the short pulse laser beam comprises a nanosecond pulse laser beam, a pulse width of the short pulse laser beam is between 1 nanosecond (ns) and 500 ns, a wavelength of the short pulse laser beam ranges from 800 nanometers (nm) to 1100 nm, a pulse energy of the short pulse laser beam ranges from 20 nanojoules (nJ) to 2000 nJ, the output power of the short pulse laser beam is between 10 watts (W) and 200 W, and the repetition rate of the short pulse laser beam is between 0.1 megahertz (MHz) and 5 MHz.

10. The apparatus (1) according to any one of claims 8 to 9, **characterized in that** the laser device (3) further comprises a vibrating mirror disposed between the laser source (100) and the optical lens set (200), the biological sample (302) is located at a fixed point in a flow channel (300) when the motion state is at a static state, and the vibrating mirror is configured to emit the short pulse laser beam onto the biological sample (302) via the optical lens set (200) in a two-dimensional or three-dimensional scanning mode.

11. The apparatus (1) according to any one of claims 8 to 9, **characterized in that** the flow rate and flow volume controller (6) is coupled to an analysis cartridge (2), and which is configured to receive the third signal to inject the biological sample (302) into a flow channel (300) of the analysis cartridge (2), and is configured to drive the biological sample (302) to flow at a flow rate ranged between 0.01 milliliter per minute (ml/min) and 1.5 ml/min in the flow channel (300) having a depth of 0.01 millimeter (mm) to 3 mm and a width of 0.01 mm to 3 mm to be treated with the short pulse laser beam along with the flow.

12. The apparatus (1) according to any one of claims 8 to 11, **characterized in that** a spot size of the focus (107) of the short pulse laser beam is between 0.1 mm and 5.1 mm in diameter, and the semiconductor laser comprises a laser diode.

13. The apparatus (1) according to any one of claims 8 to 12, **characterized in that** the optical lens set (200) comprises a light receiving lens (210) and a focusing lens (230), wherein a numerical aperture of the light receiving lens (210) is ranging from 0.2 to 0.55 and a numerical aperture of the focusing lens (230) is ranging from 0.2 to 0.55.

14. The apparatus (1) according to claim 13, **characterized in that** the light receiving lens (210) comprises a short-focus lens, and the focusing lens (230) is selected from a group consisting of an aspheric lens, an objective lens, a spherical lens, a diffractive optical element and a non-curved lens.

15. The apparatus (1) according to any one of claims 8 to 14, **characterized in that** the flow rate and flow volume controller (6) is selected from a group consisting of a syringe pump, a cylinder-type power pump, a quantitative liquid pump and a micro gas pump.
